# EUROPEAN PATENT APPLICATION

(11) **EP 1 892 525 A1**
(43) Date of publication of application: **27.02.2008**
(21) Application number: 06253785.7
(22) Date of filing: 19.07.2006
(51) Int. Cl.: G01N 29/06, G01N 29/34, G01N 29/07, G01N 29/50, G01N 29/44, G01N 29/265, G01N 33/38

(54) **Hand-held ultrasonic inspection device with wireless communication means**

(30) Priority: 20.07.2005 GB 0514920
(71) Applicant: Andrews, David Richard, Cambridge CB4 5NX (GB)
(72) Inventor: Andrews, David Richard, Cambridge CB4 5NX (GB)

(57) **Abstract**

An inspection device with lightweight, hand-held devices uses sound waves or ultrasound waves to inspect the interior of structures under test and possibly to generate images of the interior, with particular application to heterogeneous materials, such as concrete. Images of the interior of the structure under test are created by repeatedly combining signals from transmitters and receivers at a plurality of different locations to reduce the effect of random scattering from the grain particles in the heterogeneous material. The device includes means for communicating between the hand-held devices without electrical cables, so that tests can be done quickly and consequently at low-cost with means to process echo-signals to create image.

## Description

The present invention relates to a device for inspecting the mechanical integrity and operational worthiness of structures, particularly structures made of heterogeneous materials, for example: concrete structures, cast iron components and austenitic stainless steel components. Examples of large structures that can be inspected are: bridges, power stations and dams.

Large structures are expensive to build and they are generally designed to last between twenty years and one hundred years. Many structures of this kind have been built since 1950 and owners and operators of these structures have a strong financial incentive to prolong using them and to avoid demolishing them when they reach the end of their design-life. There is also a significant environmental cost associated with demolishing and rebuilding which it is desirable to avoid. Consequently, it is desirable to prolong using structures of this kind for as long as possible.

It is also desirable to use structures with reduced material quantities because this brings down the cost of construction, however, reduced material quantities can result in lower margins of safety.

When using a structure beyond its design life or with lower safety margins it becomes particularly important to inspect and to monitor its mechanical integrity and to review its operational worthiness more closely in order to maintain safe operating conditions. It is also financially advantageous to direct maintenance and repair to locations with the most important or significant deterioration.

Clearly the quality of inspection is important when reviewing the operational worthiness of ageing structures and lightweight structures. Current inspection procedures typically involve a visual examination by an experienced structural engineer. Many forms of deterioration of structures are invisible from the surface of a structure, for example the corrosion of steel tendons in post-tensioned, reinforced concrete structures. Many experts consider visual inspection to be inadequate.

It would be desirable if an inspection device could measure or reveal the following information in concrete structures: the speed of sound in transmission and from experimental data infer the strength of the material of which the structure is made, the existence of any delamination cracks, the thickness of beams and images of the interior components. It would be particularly desirable if the inspection device could be used in the form of two or possibly more roving, hand-held devices, able to be used quickly and easily together, able to operate substantially without cables yet still able to communicate information between the hand-held devices and preferably able to communicate with an ancillary image processing device, such as a mobile computer.

### STATE OF THE ART

Structures made from steel reinforced concrete get most of their strength from the steel forms therein, particularly any steel tendons, and the concrete exists partly to protect the steel from corroding due to the environment. Since the steel forms are embedded in concrete the steel cannot be inspected visually. Clearly, there is a need to inspect concrete structures to verify the strength of the concrete and the integrity of any steel forms therein.

It is known to inspect or monitor concrete structures by a number of methods: thermography, gamma ray, X-radiography, vibration holography, neutron radiography, dye penetration, magnetic induction, electric potential mapping, radar, acoustic emission, ultrasonic time of flight, ultrasonic resonance spectroscopy, ultrasonic pulse-echo, ultrasonic guided-wave, and electromagnetic guided-wave testing. Many of these methods have disadvantages; for example, radar, or microwaves, cannot penetrate the metal duct surrounding post-tensioned tendons. X-rays can penetrate the tendon duct and detailed images of steel forms in concrete can be made but exposure times for creating an image are typically several hours and it may not be possible to keep the structure fully operational during exposure because of the radiation hazard inherent with X-rays.

Sound waves and ultrasonic waves, referred to herein collectively as sound waves, are inherently sensitive methods for probing virtually all structural materials, including concrete. Because they are mechanical waves, sound waves are intrinsically sensitive to the mechanical composition of any material; by way of contrast, electromagnetic waves are sensitive to changes in the electrical properties of materials and may, therefore, only be indirectly sensitive to mechanical properties. Sound waves are known for testing steel and many other metals but these are generally, substantially homogenous materials. Concrete is a heterogeneous material, it is not a homogeneous material so conventional sound wave methods, using substantially plane waves and phase-sensitive, coherent transducers, do not work well on concrete.

It is known to test a range of materials using sound or ultrasound using one or two and sometimes more electro-mechanical conversion devices referred to herein as transducers, by which means sound waves or ultrasound waves are first caused to be emitted into the material under test using a transmitter transducer and sound or ultrasound waves in the material are collected by a receiver transducer and converted into an electrical signal which is displayed to an operator. It is known to test heterogeneous materials, materials with a significant internal grain structure, such as concrete with large aggregate constituents, using ultrasonic pulse-echo equipment with two ultrasonic transducers frequently made with relatively large apertures used in an A-scan test, with an amplitude against time representation of the received signal; in this case the transducers are made large and/or resonating in an attempt to collect more ultrasonic energy. However, the quality and quantity of information from these tests is generally poor and they are of limited usefulness. The roughness of most concrete surfaces results in relatively few points of contact across the apertures of the transducers, which itself results in a significant reduction of energy transmitted and received. It is also possible to use the impact of a ball onto the concrete surface as an alternative transmitter and to use a small, point-contact receiver but the usefulness of the test remains substantially less than inspection engineers would like.

Many transducers used in testing heterogeneous materials using sound or ultrasound waves share a common feature that they combine the signals from ultrasonic waves over the relatively large area of the aperture. This works well when a wave with plane wave-fronts is received parallel to the plane of the transducer. However, plane waves are seldom received in heterogeneous materials even if plane waves are launched by the transmitter or transmitters. The reason plane waves cannot exist in heterogeneous materials is because the randomly distributed grains, which are the important characteristic of heterogeneous materials, cause random scattering of the sound or ultrasound waves passing through them and this results in partial disruption of the coherence of the ultrasonic wave-fronts. What arrives at the transducer is not a plane wave-front but a partially randomized pulse of spherical wavelets with partly random phase differences. This scattering is only significant if the wavelength of the sound wave or ultrasound wave is commensurate with or smaller than the largest grain size. All materials become heterogeneous if the wavelength is sufficiently small in this sense and, consequently, the frequency is sufficiently high. Many materials can be tested adequately away from this condition and then the materials appear homogeneous and random scattering is not a significant effect.

It is common in the ultrasonic inspection industry to use a phase-sensitive receiver of either a single large aperture receiver (larger than the wavelength) or an array of point receivers, with a size over the array greater than one wavelength, with a fixed phase-sensitive combination of signals, frequently in the form of simple addition of signals. However, when the material is behaving heterogeneously, randomly ordered wavelets arrive at phase-sensitive receivers and try to cause randomly localized electrical charges of random polarity and values of which combine destructively within the receiver, resulting in a signal level much smaller than would be achieved with plane waves parallel to the plane of the receiver. In an extreme case, it is possible for the output signal of a phase-sensitive receiver to have zero amplitude, despite the arrival on it of a powerful but disrupted packet of sound waves. Moreover, the larger the aperture or the more transducers in a phase-sensitive receiver the smaller the signal will probably be because there is a greater probability of large phase differences, resulting in almost total destructive interference. The inspection device described herein avoids this problem by using a transmitter and receiver, each with an aperture no greater than the wavelength of ultrasound used in the test, ensuring minimal phase cancellation at each receiver.

Examples of materials that are heterogeneous under the conditions for which inspection is desirable using sound or ultrasound waves are: concrete, cast iron and austenitic stainless steel.

It is known to test concrete in a transmission test, with a transmitter and receiver on either side of parallel-sided sample, so that ultrasonic and/or sonic waves travel from the transmitter through the concrete and into the receiver. By using a sharp electrical excitation source or spike applied to the transmitter and a synchronized receiver and circuit, it is possible to measure the time between when the spike is emitted and when the first ultrasonic or sonic energy is received, from which measurement the speed of sound can be calculated provided the distance travelled through the concrete sample by any sound waves or ultrasound waves is known, the speed being given by dividing the distance by the time. The strength of concrete so tested can be inferred from the speed measured, using results of calibration tests in which the speed of sound is measured in controlled concrete test samples of known but different concrete strengths. This test forms a part of some national and international standards. The method measures the time of arrival of the first sonic or ultrasonic energy, all subsequent ultrasonic and sonic waves are ignored. One disadvantage of this method is that it does not measure the amplitude of the pulse of waves collected by the receiver that was caused by the pulse emitted by the transmitter, generally because the transmitted pulse is very long and it is difficult or impossible to identify any pulse clearly, consequently, it is impossible to use data from this test to make an attenuation tomography image of the sample, which would otherwise be desirable.

It is also known to test concrete in a reflection test, with a transmitter and receiver on the same side of the sample, so that ultrasonic and/or sonic waves travel from the transmitter into the concrete and echoes return to the receiver. The impact of a small steel ball onto the surface of the concrete is sometimes used as an alternative transmitter of sound waves and ultrasound waves and a receiver sensitive to a relatively wide frequency range is used to collect echoes from the concrete. In this way it is possible to record the variation of electrical signal from the receiver with time, over a period of roughly 1 millisecond; it is then known to take the Fourier transform of the recorded signal and thereby display the magnitude of the amplitude against frequency instead of time. Any peaks in the frequency domain representation are assumed to be due to reverberations in resonators in the concrete and the presence of these peaks is used to infer, in particular, the existence of delamination cracks, which can form resonators with the surface of the concrete. Delamination cracks can be caused by severe corrosion of the outermost network of steel reinforcement bars, which swell as a result of corrosion, putting the concrete between the bars into a state of tension that can cause micro-cracks to form in the concrete and it is the coalescence of many micro-cracks that results in a large delamination crack. This method of using ultrasound to detect delamination cracks is incorporated into some national and international standards.

It is known that many ultrasonic inspection devices apply a sharp high voltage spike to a transmitter thereby causing the transmitter to launch an ultrasonic wave of short duration; nearly all commercial ultrasonic inspection systems try to keep the ultrasonic wave pulse as short as possible. The transmitted wave pulse is generally reflected within the sample under test with an echo returning to a receiver. When this echo rises above a fixed threshold it is judged to be significant for the purpose of interpretation. Experience shows that fixed threshold levels do not give good results with heterogeneous materials, in the presence of random scattering by grains, because those grains close to the surface and immediately under a transmitter invariably contribute a stronger echo than more distant reflectors so that the operator has a problem to detect echoes of interest. Spike excitation has a number of other disadvantages: the energy efficiency of creating sound or ultrasound waves is poor because much of the energy in the spike is outside the frequency range of sound or ultrasound waves that can be generated by a transducer; consequently, unused energy in the spike can generate radio frequency interference; relatively low signal-to-noise ratio in any receiver signal, since it is the energy in the ultrasonic wave that contributes to the signal-to-noise ratio. Other forms of excitation are possible that do not have these disadvantages but which require more sophisticated signal processing of received signals, for example, swept-frequency chirp signals which are used extensively in radar systems but seldom in ultrasonic inspection systems.

A sound wave in a solid can be of two main types: compression and shear. A compression wave travels with particle motion parallel to the direction in which the wave is moving; a shear wave travels with particle motion perpendicular to the direction in which the wave is moving. There is only one direction of particle motion possible for a compression wave, it is sometimes known as a scalar wave for this reason, but for a shear wave the direction of particle motion can be at any angle to the direction of the wave so it is common to refer to shear waves as vector waves. A shear or vector wave has a direction of polarization referred to the direction of any two, convenient, perpendicular vectors, each of which is perpendicular to the direction of the wave. It is also possible to have waves associated with surfaces and hybrid waves which are a collection of more than one fundamental type of wave. By way of contrast, liquids only support compression, not shear, so in a liquid there are only compression waves.

It is known that different types of waves travel at different speeds, particularly compression and shear waves and it is found that shear waves generally have a wave speed of roughly 60% of the speed of compression waves. The existence of these different wave types presents a difficulty for the inspection of solid materials with sound waves because, for example, a crack in a metal can reflect a compression wave and generate a shear wave at its tip, thereby generating two waves from approximately the same place that will travel back to a receiver. If the receiver transducer were sensitive to both compression and shear then there would be two peaks in the electrical signal from the receiver; this may be acceptable when testing a single flaw in an otherwise pristine sample, with no other echoes, but the presence of even a few other echoes quickly makes the situation confusing for the operator: which echoes come from the same reflector or flaw? In most commercial inspection systems the situation is simplified by using a single transducer to both transmit and receive that is more sensitive to one of the wave types than to the other. Information from the other wave type is ignored but this is potentially valuable information. In the inspection device described here it is possible to transmit either compression or two orthogonal polarizations of shear waves and to receive using compression or the two orthogonal polarizations; the inspection device does not discard any information and permits information from different wave types to be viewed by the operator.

It is known in the medical application of ultrasound inspection to create images based upon harmonics of the transmitted ultrasonic wave and this sometimes gives improved contrast to changes in tissue. Harmonics are created when sonic or ultrasonic waves pass through a material that is non-linear or through an interface that is non-linear, such as a crack. A crack will support the compressive half-cycle of a sound or ultrasound wave but may not support the tensile half-cycle, which causes cracked materials to be non-linear materials and to result in harmonic and possibly sub-harmonic generation of sound and ultrasound waves.

### STATEMENT OF INVENTION

Accordingly, in a device for inspecting the mechanical integrity of structures, using sound waves or ultrasound waves, there is provided: (a) at least one and, preferably but not necessarily, two or more mechanically independent hand-held devices each containing one or possibly more sonic and/or ultrasonic electro-mechanical conversion transducers; (b) means for each aforementioned hand-held device to communicate with other hand-held devices, substantially without the use of cables, so that the operator or operators of the inspection device are substantially unencumbered by cables; (c) means to cause any of the transducers in either of the aforementioned hand-held devices to transmit sound waves and/or ultrasound waves; (d) means to collect electrical signals from any of the transducers, used as receivers, in the aforementioned hand-held devices, which signals are caused by the impingement of sound waves and/or ultrasound waves emerging from the structure under inspection and entering the receivers, particularly waves caused by a transmitting, hand-held device; (e) the aforementioned transducers with some or possibly all able to transmit and/or receive substantially compression sound waves or ultrasound waves, or with possibly some or possibly all of the aforementioned transducers able to generate and/or to receive substantially shear sound waves or ultrasound waves; (f) with the direction or plane of shear polarization of the aforementioned shear-transducers all being known and capable of being set by an operator; (g) each aforementioned transducer with an aperture that is preferably not larger and possibly smaller in actuating dimension than an average wavelength of sound wave and/or ultrasound wave used by an inspection device; (h) means to couple or transfer sound wave or ultrasound wave energy between each aforementioned transducer and the surface of the structure under inspection, when engaged thereupon; (i) means for creating and applying excitation patterns to transducers selected to transmit in the aforementioned hand-held devices; (j) means to synchronize the transmission of sound waves and/or ultrasound waves from one aforementioned hand-held device with the receiving of sound waves and/or ultrasound waves from another of the aforementioned hand-held devices; (k) means for collecting and storing signals received by selected transducers in one or preferably more of the aforementioned hand-held devices; (1) means in one or preferably more of the aforementioned hand-held devices for processing the signals from receivers to provide a more convenient or advantageous representation of results for the operator; (m) means in the aforementioned hand-held devices to communicate information with one or possibly more communicating devices for the purpose of improving the quality or speed of communications between hand-held devices; (n) means in the aforementioned hand-held devices to communicate information with one or possibly more processing devices for further processing or advantageous representation of results; (o) means to allow the inspection device to be used quickly and easily to inspect a structure.

### CONSISTORY CLAUSES

It is desirable that the cost of an inspection at any location on a structure should be kept as low as possible; one way to help to achieve this is if the inspection at any given location is done quickly. The time to move between one location and the next should be as short as possible and one way to ensure this is to have as few electrical cables as possible. Consequently, it is desirable that the inspection device should use radio waves or light waves to communicate between the hand-held devices in an inspection device.

The geometrical layout and construction of a structure could make it difficult to communicate effectively between hand-held devices, for example a large, heavily-reinforced concrete structure can block radio waves and it is opaque to light. It is desirable under these conditions to use one or possibly more communicating devices as repeaters between the transmitter device and the receiver device.

Another way to keep the time to inspect as short as possible is to make the hand-held devices in the inspection device portable and lightweight.

Another way to keep the time to inspect as short as possible is to make the inspection device capable of performing tests quickly and capable of working continuously for several hours.

It is preferable but not essential to complete testing at a location, to process the resulting signals and display the results to the operator or inspection engineer within a few seconds so it is desirable to use fast electronic devices in an inspection device, particularly using a digital representation of signals.

To simplify the interpretation of received signals in an inspection device it is preferable but not essential to transmit either substantially compression-waves or substantially shear-waves and to receive either substantially compression-waves or substantially shear-waves. In this way the inspection device can be operated in as many as nine possible inspection modes according to wave-type: transmitting compression waves and receiving shear parallel waves, transmitting compression waves and receiving shear perpendicular waves, transmitting compression waves and receiving compression waves, transmitting shear parallel waves and receiving compression waves, transmitting shear parallel waves and receiving shear perpendicular waves, transmitting shear parallel waves and receiving shear parallel waves, transmitting shear perpendicular waves and receiving compression waves, transmitting shear perpendicular waves and receiving shear parallel waves and transmitting shear perpendicular waves and receiving shear perpendicular waves.

It is preferable but not essential for each hand-held device in an inspection device, either transmitter or receiver, to be equipped with two transducers: one compression and one shear, with the shear transducer having a mark on it to indicate the plane of polarization of shear or a mark on the case of the hand-held device or in any other way to indicate the plane of polarization of shear. One way to mount the transducers conveniently is to place the compression transducer at one end of a hand-held device and the shear transducer at the other end of the same hand-held device so that the operator simply has to turn around a hand-held device to change between compression and shear testing. Another way to mount the transducers is to have two transducers, one compression and one shear, on a turret so that the appropriate transducer can be rotated into place. However the transducers are mounted, it is essential that the choice of transducer, either transmitter or receiver, made by the operator is signalled to electronic circuits to which the transducers are connected in the hand-held device; one preferred means is to use a micro-switch or a similar device to sense when a transducer is being pressed against the sample under test; in the case of turret-mounted transducers the position of the turret can be used to indicate the transducer.

It is desirable that an inspection device can be used in any one of a plurality of different modes, including but not necessarily limited to: transmission, transmission for tomographic imaging, reflection, reflection for synthetic aperture imaging; with the above modes compounded when the plurality of options for compression and shear are also included. In the two imaging modes it is important that the position of the transducers is known so that the algorithms for generating images can function correctly. One way that positions can be defined and position information can be obtained or defined or controlled is if a thin, flexible belt or sheet with holes arranged randomly spaced in one dimension or two dimension or in some other known desirable, semi-random pattern is attached to the surface of the structure to be inspected, through which holes the transducers of any hand-held devices are inserted by an operator and thence come into contact with the surface of the structure. It is desirable that the holes are not arranged periodically, such on a regular grid or with parallel lines, because the spatial Fourier transform of a periodic arrangement like this has a periodicity which can enter into the image and cause distortions or artefacts, so a random or randomized but known pattern, which does not create periodic peaks in the spatial Fourier transform, is preferable. It is desirable that the procedures or algorithms that generate images have information about the belt or sheet, particularly the positions of the holes therein, and information about the geometry of the part of the structure being imaged then it is possible for the algorithms and the operator working together to control the positions of the transducers for experimental procedure to be efficiently managed and for correctly calculated images to be generated.

Reflected signals received from transducers at two or preferably more positions by an inspection device can be usefully combined in an image to reduce substantially the otherwise obfuscating effect of random scattering of sound waves or ultrasound waves, this is a form of what is sometimes called spatial averaging. A preferred but not essential method for applying spatial averaging and creating an image is called synthetic aperture focussing, which has a focal plane chosen by an operator, divided conceptually into a plurality of focal points, possibly again chosen by the operator, and for each focal point in the focal plane as many stored echo signals as possible or desirable are processed by adding appropriate parts of them together with suitable time-delays chosen to compensate for the geometrical distances travelled to create an intensity at each focal point. By repeating the process for every focal point in the focal plane an image is developed over the entire focal plane which is relatively sharp. By setting a focal plane inside the structure and at different depths or orientations an image of the interior of the structure can be viewed by the operator.

Transmitted signals received by transducers at two or preferably more positions through a cross-sectional plane of a structure by an inspection device can be usefully combined in an image to reduce substantially the otherwise obfuscating effect of random scattering of sound waves or ultrasound waves, this is another form of what is sometimes called spatial averaging. A preferred but not essential method for applying spatial averaging and creating an image is known as tomographic imaging, which has an image plane defined by the locations of the transmitters and receivers, divided conceptually into a plurality of image points defined by the number and locations of the transmitters and receivers; at each image point some degree of attenuation and possibly scattering of sonic or ultrasonic signals is assumed to occur and by taking the path of waves followed in each transmitter-receiver test in turn it is possible to calculate the degree of attenuation and/or the degree of scattering at each image point in the cross-section and to create an image to represent the geometry of the cross-section of the structure with the degree of attenuation or the degree of scattering represented thereupon using, possibly but not necessarily, a range of colours to represent the level of attenuation and/or scattering.

It is known for heterogeneous materials that the degree of random scattering of sound waves or ultrasound waves from grains increases as the wavelength is made as small as the grain size; it is, therefore, preferable but not essential for an inspection device to use sound waves or ultrasound waves with wavelengths greater than the grain size. By way of an example, for concrete with a largest aggregate size of 20 millimetres and a speed of sound of 4000 ms⁻¹, then the wavelength should, preferably, be greater than 20 millimetres which requires the use of frequencies less than 200 kHz because it is known that the speed of sound is equal to the product of frequency and wavelength.

The method of coupling or transferring ultrasonic energy between each transducer and the surface under test is important because it affects the quality and strength of the ultrasonic signals transmitted and received and therefore, ultimately, the quality of any results including images provided by the inspection device. The coupling method should be quick and easy to use, to keep testing times as short as possible, and to keep the cost of testing as low as possible. Point contact transducers can be used under favourable conditions, for example: a smooth test surface with little or no surface dust or friable material; but point contact transducers are intrinsically inefficient, in terms of the amount of energy that can be transferred between the transducer and the testing surface and from the test surface to the transducer consequently point-contact transducers are more likely to reverberate which implies that point-contact transducers are not generally capable of working over a wide range of frequencies. It is desirable to use transducers and signals with as wide a bandwidth as possible in an inspection device because this results in shorter pulses of waves being transmitted and received and, consequently, better axial resolution of components in the structure being inspected; therefore it is preferable to use transducers that transfer or couple ultrasonic energy more efficiently into a test sample than a point-contact transducer by using a larger aperture area than point contact.

The largest preferred aperture size is approximately one wavelength of the highest frequency of sound or ultrasound used in a test; this preference results because it is desirable to minimise phase-sensitive interference effects occurring intrinsically over a receiver. By way of an example of how to select a suitable aperture size, using concrete as an example material but extending the principle to all heterogeneous materials, it has been found that test frequencies up to about 200 kHz give good results and assuming the wave speed is 4,000 metres per second in concrete a receiver aperture size of 20 millimetres or smaller is preferred; speed is equal to the product of frequency and wavelength. A preferred embodiment of an inspection device for use on concrete has a diameter of the transducer not greater than 50 millimetres and preferably not greater than about 20 millimetres.

The surface roughness of the test surface, for example concrete, can also result in low efficiency of coupling because sound or ultrasound waves can only travel through asperities or point contacts with a small area. Certain coupling agents can be used to improve the efficiency of coupling and for the specific example of concrete a preferred coupling material is a fast-setting mortar. Solid coupling agents such as adhesives transmit compression and shear waves equally well. Another class of preferred coupling agent is thin deformable materials and a preferred deformable material to use on concrete is silicone rubber filled with mineral fillers to increase the acoustic impedance and thereby improve the efficiency of coupling. It is the relationship values of acoustic impedance at the interface between two materials that determines the degree of transmission and reflection of acoustic waves and ultrasonic waves: when the two values of acoustic impedance for the two materials are the same then there will be perfect transmission and no reflection. It is desirable that the transmitting surface of the transducer, the coupling agent and the material of the surface of the structure being inspected, all have approximately the same value of acoustic impedance.

A preferred transducer for testing structures with rough surfaces has a substantially flat actuating surface and is made of a material with an acoustic impedance matched to the material of which the structure or the surface layer of the structure is made, with a small dome protruding from the flat surface of a height a little more than the size of the surface relief of the structure being inspected. In use the protrusion can fit into a suitable valley in the surface relief and generally couple over a larger surface area of the dome than would otherwise occur with an asperity of the surface in contact with the otherwise flat surface of the transducer.

A preferred transducer specifically for testing concrete has a substantially flat surface, made of a material with an acoustic-impedance matched to concrete that adheres to mortars, with an aforementioned small dome of a few millimetres in height protruding from it. This particular design can be used: for point-contact coupling, with liquid-gel coupling materials, with filled silicone rubber coupling materials and with fast-setting mortar, so that the operator has a choice of coupling material to use to suit the prevailing test requirements and conditions of the surface to be tested.

It is preferable but not essential for each hand-held device in an inspection device to be capable of use either as a transmitter or as a receiver because this allows hand-held devices to be used interchangeably.

It is preferable but not essential for each hand-held device in an inspection device to have means to avoid using transducers simultaneously as receivers and transmitters thereby retaining maximum information from the interior of the structure under test because a single, combined transmitter-receiver cannot be receiving when it is transmitting so that during periods of transmission received information is lost.

It is preferable but not essential in an inspection device to drive transmitter transducers with bursts of electrical excitation in a controlled pattern. It is preferable but not essential to use a swept-frequency chirp. A chirp is a burst of sine waves lasting more than one cycle during which time the frequency of the sine wave is changed. For example when testing concrete a suitable chirp could last for between 30 microseconds and 300 microseconds during which time the frequency could sweep over a range of frequencies linearly in time somewhere in the range from 200 kHz to 10 kHz. For inspecting stainless steel higher frequencies and shorter chirp durations are preferably used. Chirps can be chosen to suit particular materials, by which is meant that the statistics of the size distribution of grains in the material will tend to control what is the highest frequency that can be propagated without too much scattering; the range of frequencies should otherwise be as great as possible. The lowest frequency or more importantly, the corresponding period of the lowest frequency, will tend to control the duration for the chirp, since a chirp with duration shorter than the period of the lowest frequency will fail to explore or pronounce the chirp.

It is desirable to match the frequency range of any pattern of excitation used to drive transmitters in an inspection device to the bandwidth of the transducers, because this will help to maximise the sonic or ultrasonic efficiency of the inspection device and consequently its sensitivity; it is also desirable to use a pattern that can be received without too much distortion after travelling a distance through the structure under test of interest to the operator; generally, lower frequencies have greater range but higher frequencies give better axial resolution.

When an inspection device is using a pattern of excitation of transmitters it is desirable to process signals from receivers so as to detect occurrences of the pattern, which processing should preferably convert the relatively long pattern into a shorter and sharper event signal and consequently result in better detection of peaks and hence in sharper images. One preferred method is sometimes called matched filtering; it is the optimum linear signal processing method for detecting a known pattern in noise and it can make use of a copy of the transmitted excitation pattern. It is preferable but not essential, however, to make use of part of the signal received during a calibrating test on a simple homogeneous sample, having the same or similar acoustic properties to the structural material of interest, because the original electrical excitation pattern is modified both by the transmitter as it is converted into a sonic or ultrasonic wave pattern and it is modified by the receiver as the sonic or ultrasonic pattern is collected and converted back into an electrical signal so the original excitation signal may not be an accurate copy of the pattern to be recognised in the received signal and an experimentally collected chirp is generally more accurate. The more accurately the matched filter is matched to the pattern found in signals received from the structure under test the sharper will be the event peak marking the arrival of the pattern and the more accurate will be any resulting images generated from the signals.

Even if spike excitation of the transmitters in the inspection device is used it is still preferable to use matched filtering based upon a pattern recorded in a calibration experiment, as described earlier here. With spike excitation most if not all transmitter transducers cause substantial distortion of the spike excitation because a spike covers a wide range of frequencies but the transmitter can only transmit waves in its, generally, much narrower bandwidth. The reduced bandwidth distorts the spike substantially and a pulse of waves is generated, which can still be efficiently detected by matched filtering.

It is sometimes desirable to convert signals from a bipolar form to a unipolar in an inspection device form to eliminate phase sensitive combination of signals such as subtraction and thereby reduce the obfuscating effects of random scattering in heterogeneous materials. A preferred method is to calculate the magnitude of the mathematical analytic function of the signal. Amongst other possible methods there are: simple rectification or taking the magnitude of the signal, these latter two are preferably but not essentially followed by low-pass filtering or band-pass filtering to smooth the signal. It is desirable that unipolar conversion follows matched filtering.

A preferred but not essential stage of processing after unipolar-conversion is to detect peaks in the signal, which may be caused by echoes from strong reflectors in the structure under test or may be the arrival of a pulse of waves in transmission. It is common in industrial inspection to apply a constant level threshold and to detect peaks that exceed the threshold, allowing them to pass and setting to zero values elsewhere. This method does not work well when testing heterogeneous materials in reflection, for example concrete, because the randomly arranged grains, or aggregates in the case of concrete, therein cause significant random scattering of ultrasonic waves during a test and grains or aggregates nearest to the transmitter cause the greatest degree of scattering, which tends to obfuscate echoes from other components in the concrete under test that are possibly of more interest to the operator. Instead of using a constant level threshold it is preferable to use a time-varying threshold to detect peaks of significance and a preferred time-varying threshold is based upon the statistics of the random scatterers in the material under test. It is preferable but not essential to use the Weibull or Rayleigh distributions to create time-varying thresholds for unipolar signals; furthermore by adjusting the parameters of the chosen time-varying threshold it is possible to provide a degree of control of over the height of peaks considered to be signifcant.

An optional but not essential stage of processing, occurring preferably but not essentially after pattern recognition and possibly but not necessarily after unipolar-conversion is to calculate the magnitude of the Fourier transform of the processed signal, thereby representing the signal in the frequency-domain instead of the time-domain, and to display it for the operator to determine any peaks therein, since this can be used to establish the presence of delamination cracks in the structure under inspection.

It is also preferable but not essential to add together either time-domain results or to add together frequency domain results from nearby test locations because this has the effect of suppressing the effect of random grain scattering and emphasizing the effect of extensive or systematic features, such as back-wall echoes, echoes from tendons, echoes from reinforcement bars, echoes from extensive delamination cracks.

It is desirable for an inspection device to have means to process received signals to recognize therein the pattern of any harmonics or sub-harmonics of the excitation pattern used to drive transmitter transducers in transmission or reflection tests and to use information or signals derived from harmonics or sub-harmonics in place of the signals derived from using the pattern directly. Harmonics are generated by non-linear propagation of sound waves or ultrasound waves. Some materials are naturally non-linear, providing the amplitude of the sound or ultrasound wave is sufficiently high; other sources of non-linearity are regions of materials or interfaces that are not mechanically continuous such as a crack, or a region of many cracks, which support compressive stresses in sound or ultrasound waves but not tensile stresses and, consequently, are non-linear materials. By detecting harmonics or sub-harmonics it is possible preferentially to locate or detect non-linear regions in the structure being inspected.

It is essential for the correct operation an inspection device that the start of a recording of a signal derived from a receiver transducer in a hand-held device is synchronized accurately with the excitation of the transmitter in a hand-held device, in particular synchronization should be done with low jitter or variability in the start of the recording, so that the jitter is much smaller than the period of the highest frequency transmitted by a hand-held device. In this way it is possible for timing measurements to be made accurately and it is possible for the imaging algorithms to function without errors that would otherwise be caused by high levels of jitter. Any communication method must be able to provide a frequency range or bandwidth not less than the reciprocal value of the jitter so that the synchronizing signal shall be communicated without distortion. By way of example, when inspecting structures made of concrete the highest frequency used for inspection might typically be 200 kHz, with a corresponding period of 5 microseconds. The jitter should be much less than 5 microseconds and a jitter of 0.1 microseconds should be acceptably small, consequently, the bandwidth of the communicating channel must be at least 10 MHz. With stainless steel and cast iron higher test frequencies are possible and correspondingly smaller jitters are required and communication means with wider bandwidths. When one hand-held device is both a transmitter and a receiver then synchronization can be achieved relatively easily by internal synchronizing means but otherwise, when the receiver is in a different hand-held device to the transmitter with no cable connection it is more difficult to achieve synchronization. It is possible to use radio signals to achieve synchronization with low jitter when the transmitter and the receiver are in different hand-held devices but it is difficult to achieve unless a bandwidth of 10 MHz or more is available on the communication channel; due to the restriction on communication bands placed by legislation in many countries this bandwidth is not readily available except at very high frequencies but high frequency radio waves do not propagate far with the low transmitter power permitted in hand-held devices, particularly in concrete structures. Communication means using light waves similar electro-magnetic waves are unrestricted by legislation and a high bandwidth is available. Consequently, it is preferable but not essential to use light or similar electro-magnetic wave communications for synchronization between two different hand-held devices in an inspection device. Another possible means for synchronizing is to make use of the natural electro-magnetic wave generated when the transmitter is actuated; the electro-magnetic wave launched has approximately the same pattern as the electrical drive signal to the transmitter transducer in the transmitting hand-held device so it is possible for the receiving hand-held device to be triggered from this signal, particularly if pattern-recognition processing is used to process the received signal, resulting in a sharper event pulse with lower jitter, however, the bandwidth available is insufficient to ensure sufficiently low jitter and it is desirable but not essential to repeat the inspection several times and take the average values of any results to reduce the degree of error thereupon.

It is possible to use two or possibly more hand-held devices, referred to here as hand-held device A and hand-held device B, in an inspection device without using electro-magnetic waves of any kind for the synchronization described above, instead a ping-pong mode of operation is used. Ping-pong mode is not guaranteed to provide low-jitter signals and consequently it may give timing measurements higher errors and poorer image quality, particularly in reflection-imaging, it may also be restricted in the range of wave-type modes that can be used. However, ping-pong inspection can be used even when other forms of communications cannot be established between two hand-held devices, thereby permitting a test to be done under adverse conditions of communication. In the ping-pong mode of operation, hand-held device A transmits a known pattern of sound waves or ultrasound waves into the structure being inspected and also starts recording possibly at the same time but preferably at some known, delayed time, of low jitter, thereafter; the delay being chosen generally to avoid recording while the transducer is transmitting. The transmitted sound or ultrasound waves from device A travel through the structure to the receiver of a hand-held device B which is set-up to use its own pattern recognition processing means to detect occurrences of the transmitted pattern of waves from hand-held device A while sound or ultrasound waves are arriving and not after making a recording of the waves. When the receiver of hand-held device B detects the transmitted pattern it stops receiving and changes to transmitting, preferably but not necessarily transmitting a different pattern of excitation compared to that transmitted by hand-held device A, possibly transmitting a time-reversed version of the pattern transmitted by hand-held device A. The re-transmitted wave from hand-held device B then re-traces the path already taken, going back to hand-held device A, now working as a receiver, where the pattern is collected, recorded and processed to provide results. It is desirable to use different patterns of transmitted waves from the two hand-held devices because otherwise hand-held device A would not be able to distinguish between echoes of its own pattern of waves caused by the structure and the pattern transmitted by hand-held device B and this would probably cause confusion and possibly large errors in times of transmission. It is more complicated to measure the time taken by waves to travel through the structure in this ping-pong mode because as well as any possible delay in recording in hand-held device A there may also be time-delays introduced at hand-held device B which should not be included in any time used to calculate the speed of sound. However, a correcting experiment can be performed with the two hand-held devices, A and B, with the transducers of the two hand-held devices touching directly or on either side of a standard block of known time-delay, to measure the time delays and these can be subtracted from the total time measured in subsequent inspection tests. After subtracting time delays the resulting time must be divided by two to get the time taken to pass once through the structure distance then speed is the distance travelled by the waves divided by the final, corrected time result. Amplitudes measured in ping-pong experiments, which may be used in tomographic imaging, do not need to be corrected for the ping-pong nature of the test. It is desirable but not essential that the hand-held devices contain means to perform correction for time-delays. It is preferable but not essential to repeat the ping-pong experiment several times and to take the average result because this helps to reduce the error in any measurements made in the ping-pong mode.

It is preferable but not essential that there is one or possibly a plurality of data processing devices in the hand-held devices of an inspection device, preferably but not necessarily: a computer or a microprocessor or a digital signal processor or a microcontroller or combinations of the aforementioned devices along with suitable memory and other electrical circuits. The data processing devices, preferably but not necessarily, control some or all of the following functions: the transmission of any sound and ultrasonic waves, the receiving of any sound or ultrasound waves, the conversion of any received waves into electrical signals, recording of received electrical signals, the processing of received electrical signals, the communications with other hand-held devices, the communications with communication devices, the communications with other processors, the creation of images, responding to button presses by the operator and any other procedures required to allow the inspection device to function.

It is desirable but not essential for the electronic circuits and other sensitive components in each hand-held device of an inspection device to be held inside an enclosure, one enclosure for each hand-held device, preferably but not essentially an enclosure sealed against moisture, humidity, dust and other environmental agents that might disturb the normal operation of the inspection device.

The hand-held devices in an inspection device, preferably but not necessarily, each contain a source of electrical energy, such as a re-chargeable battery or batteries, which can conveniently reside with the majority of the electrical circuits comprising the signal processing unit inside an enclosure. Each hand-held device is, preferably but not essentially, sufficiently lightweight to be carried by the operator in the operator's hand for periods of hours. This arrangement helps to reduce the time spent on each test and helps to keep down the cost of an inspection.

It is also preferable but not essential for the operator to be able to control a test using switches and similar devices preferably but not necessarily mounted on each hand-held device in an inspection device in conjunction with information on a display panel mounted preferably but not necessarily on each hand-held device.

It is preferable but not essential for the inspection device to have means in one or more hand-held devices to calculate the speed of sound or ultrasound of compression and/or shear waves in the structure under test. It is preferable to use the inspection device on a part of the structure with a known dimension and to have means to enter the value of the dimension into hand-held device so that the calculation of speed can be done.

It is preferable but not essential that the display panel on a hand-held device, forming part of an inspection device, should not be too large and not too heavy so that each hand-held device is small enough and light enough to be held by hand. Due to these restrictions on its size, it is possible that the display panel may not be sufficiently detailed for images generated by the inspection device to be viewed on the display panel and, therefore, an ancillary display panel is desirable. It is also possible that a faster processor is needed for image generation than can be used in a hand-held unit or that more memory is needed and so, for certain embodiments of an inspection device, it is desirable to transfer signals, possibly but not necessarily, after some processing, to an ancillary processor in a processing device, preferably but not necessarily a mobile computer, for further processing, storing of information and better to display images.

It is also possible for all data entry and control to be entered into an ancillary device, possibly but not necessarily, a mobile computer and for the ancillary device to communicate the settings and data to the hand-held devices forming the inspection device.

It is desirable that the transducers on a hand-held device, forming part of an inspection device can be detached and replaced but otherwise held in place by mechanical means. The advantage being that different transducer types can be used in this way or that servicing of the hand-held device can be more easily accomplished.

### SPECIFIC DESCRIPTION

Specific embodiments, L, M, N, of the inspection device will now be described for use in various inspection modes; two specific embodiments of hand-held devices, A and B, will now be described each of which can be used in L, M and N.
Figure 1 shows a sketch of embodiment A of a hand-held device with fixed transducers, one at each end of the hand-held device.
Figure 2 shows a sketch of embodiment B of a hand-held device with transducers on a rotating turret, all at one end of the device.
Figure 3 shows a sketch of two of hand-held devices, each of embodiment A, used together with a mobile computer and a communicating device as embodiment L of an inspection device, all in use on a test sample.

Referring to drawing 3, the inspection device comprises: a pair of hand-held devices, 10, held in contact onto a sample to be inspected, 9, with a communicating device, 11, to assist in passing communicating signals between the two hand-held devices, 10, with the communicating device, 11, positioned by the operator to improve communications. The hand-held devices, 10, and the communicating device, 11, communicate using either radio with antenna, 4, or by optical waves, with an optical transceiver, 5, and by the same communication means with a mobile computer, 16, having a display panel, 17.

Referring to drawings 1 and 2, each hand-held device has: a sealed case, 8, with electronic circuits therein; a compression transducer, 1, a shear transducer, 2, the latter with a mark, 19, on the case, 8, to indicate the shear plane orientation; coupling layers, 3, between the transducer and the structure under inspection; a display panel, 6; buttons, 7, with electrical switches under to allow the user to control the inspection device; a radio antenna, 4, and an optical communicating transceiver, 5; in embodiment B a turret, 12, for rotating transducers into place.

Referring to drawings 1, 2 and 3, using either embodiment A or B of a hand-held device in embodiment L of an inspection device it is possible to use an ancillary computer, 16, with a display, 12, radio antenna, 4, and optical transponder, 5, with the hand-held devices for running computationally intensive imaging algorithms.

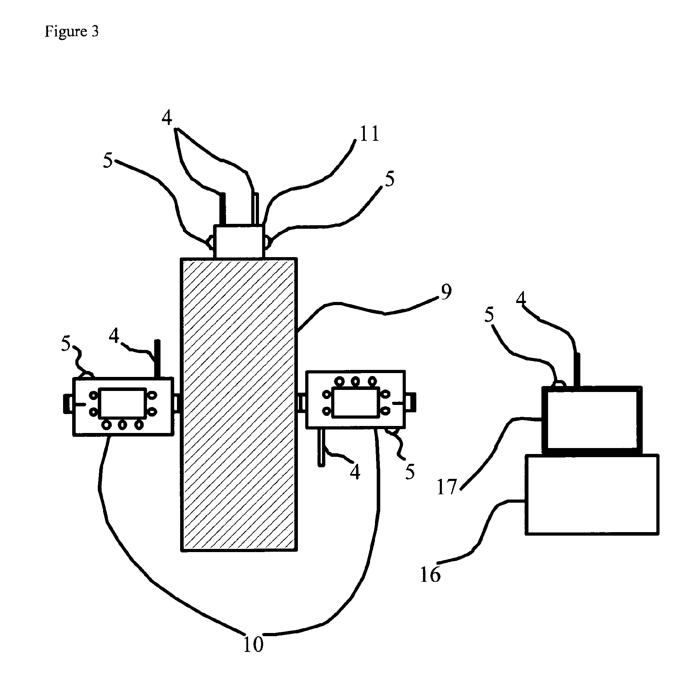

In use the operator first switches on both hand-held devices, 10, using one or possibly more of the keys, 7. The operator sets the mode of operation for the inspection device, which involves selecting a suitable transducer, 1 or 2, for each hand-held device and some pressing of keys, 7, so that the controlling program running on a processor inside each hand-held device can be set to operate in any mode chosen by the operator. The operator then presses each hand-held device, 10, against the structure, 9, being inspected and causes a transmission test to be performed, as shown in figure 3 but the same steps are followed when performing a reflection test or an imaging test using either transmission or reflection, taking care when using shear transducers, 2, to maintain the correct plane of polarization. The two hand-held devices, 10, communicate and synchronize the operation of their programs; as the transducers are pressed against the surface a micro-switch within each device is actuated and when the micro-switches in both the transmitter hand-held device, 10, and the receiver hand-held device, 10, are both actuated the transmitter transducer is excited with a swept-frequency chirp, which launches a known pattern of sound waves and/or ultrasound waves into the sample under test. As the waves are launched, or approximately at that time, the optical light transceiver, 5, communicates a short synchronizing pulse to the second hand-held device so that the receiver circuits therein start to record. The sound and/or ultrasound waves travel into the sample and are reflected and transmitted by the components therein until some waves arrive at the receiver transducer, are converted to electrical signals, then amplified and recorded. Shortly after recording has ended, the signals are processed to recognize the pattern of the linear-swept frequency chirp. In a transmission test unipolar conversion is performed and the operator can use the keys, 7, to enter the thickness of concrete through which the sound waves and/or ultrasound waves have travelled; the processor or processors in the electronic circuits inside the hand-held devices then calculate the speed by finding the time taken for the first significant peak of energy to arrive and then calculating the speed by dividing the distance the sound or ultrasound waves travelled in the test sample by the time of arrival of the peak, showing the result on the display, 6. If a reflection test is performed the operator can select, using the keys, 7, for a Fourier transform to be done and to show the results on the display, 6. It is also possible to show the processed signals on the display.

Figure 3 shows embodiment L used in transmission mode.

When embodiment L is used in reflection mode instead of transmission the only change is that both hand-held devices are used substantially from the same surface.

Embodiment, M, of the inspection device will now be described used for tomographic imaging; embodiments A or B of the hand-held devices could equally well be used in this embodiment.

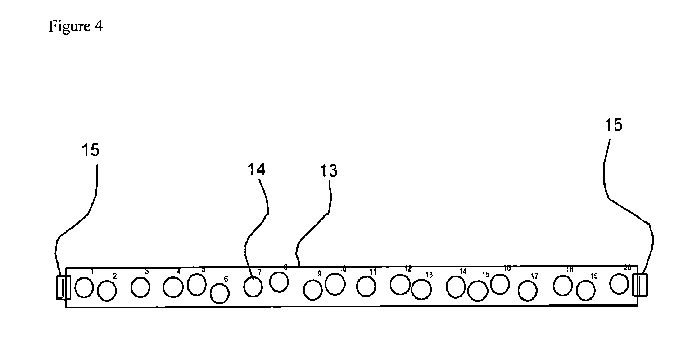

Figure 4 shows a sketch of a belt, used to define the cross-section to be used for tomographe imaging in embodiment M of an inspection device.

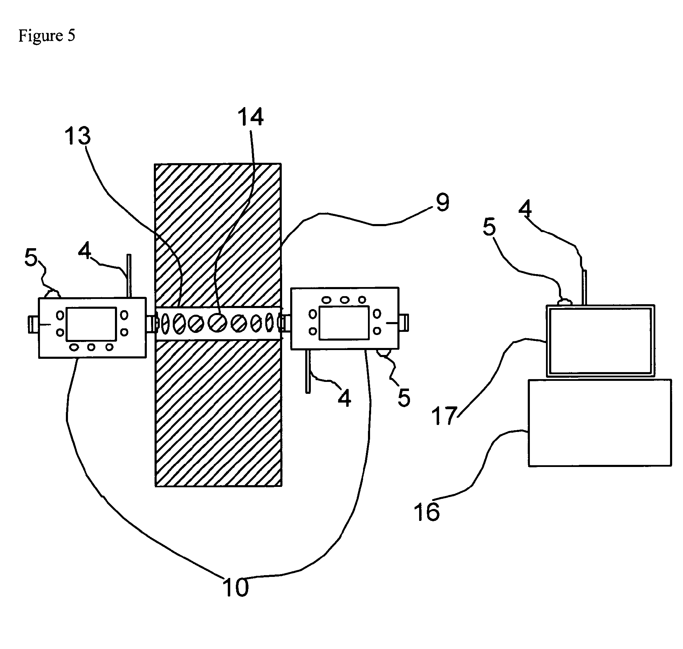

Figure 5 shows a sketch of two hand-held devices and a belt in use during a tomographic imaging inspection in embodiment M of an inspection device.

Referring to drawings 1, 2, 4 and 5 the belt, 13, is attached temporarily around a test structure, 9, to be imaged; a cylindrical pillar is shown here as the structure under inspection only for the sake of an example, any shape permitting access around all of it or most of it can be inspected. Clips, 15, at each end of the belt can be used to hold the belt in place but otherwise a temporary weak adhesive can be used to hold the belt in place on the structure. Transducers on the hand-held devices, 10, are pressed through the holes, 14, in the belt, 13, onto the surface of the structure being inspected, 9. Holes in the belt, 14, are marked with identifying labels and the controlling program identifies these labels; the display on each hand-held device shows the label for the next hole to use. In embodiment M it is also possible to use an ancillary computer, 16, with a display, 17, radio antenna, 4, and optical transceiver, 5, with the hand-held devices, 10, for running computationally intensive imaging algorithms.

In use the operator fixes the belt in the correct position on the part of the structure to be imaged and starts the mobile computer, 16, and its imaging program, entering data on the geometry of the structure under the belt, 13. The operator starts two hand-held devices working in an appropriate mode by pressing keys, 7, and selects a transducer type to use on the two hand-held devices: compression or shear, and presses the appropriate transducer on each hand-held device, 10, against the structure, 9, in holes, 14, on the belt, 13, indicated by the display panel, 6, on each hand-held device thereby performing one transmission test, taking care when using shear transducers to maintain the correct plane of polarization. As the transducers are pressed against the surface a micro-switch within each device is actuated and when the micro-switches in the transmitter hand-held device, 10, and the receiver hand-held device, 10, are both actuated the transmitter transducer is excited with a swept-frequency chirp, which launches a known pattern of sound waves and/or ultrasound waves into the sample under test. As the waves are launched by the transmitting hand-held device, or approximately at that time, the optical light transceiver, 5, sends a short, synchronizing pulse causing the receiving hand-held device to start to record. The sound and/or ultrasound waves travel into the sample and are reflected and transmitted by the components therein until some waves arrive at the receiver transducer, are converted to electrical signals, amplified and recorded. Shortly after recording, the signals are processed to recognize the pattern of the swept frequency chirp. At this point the signal is either stored or, preferably but not necessarily, communicated by radio, 4, or optical waves, 5, to a nearby mobile computer, 16, upon which the tomography algorithm is operating; the image is presented on the display, 17, associated with the mobile computer.

Embodiment, N, of the inspection device will now be described used for reflected wave imaging; embodiments A or B of the hand-held devices could equally well be used in embodiment N.

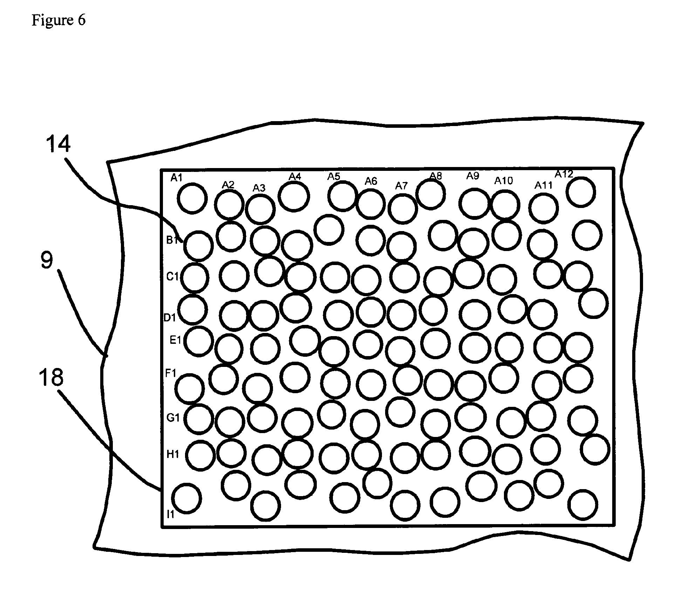

Figure 6 shows a sketch of a flexible sheet, used to define the positions of transducers to be used for reflection imaging attached to the location of interest, 9, on the structure being inspected. The flexible sheet is used with two hand-held devices and a mobile computer in substantially the same ways as already shown in figures 3 and 5.

Referring to figure 6 there is a lightweight, thin flexible sheet, 18, with a plurality of holes, 14, each identified by a reference label.

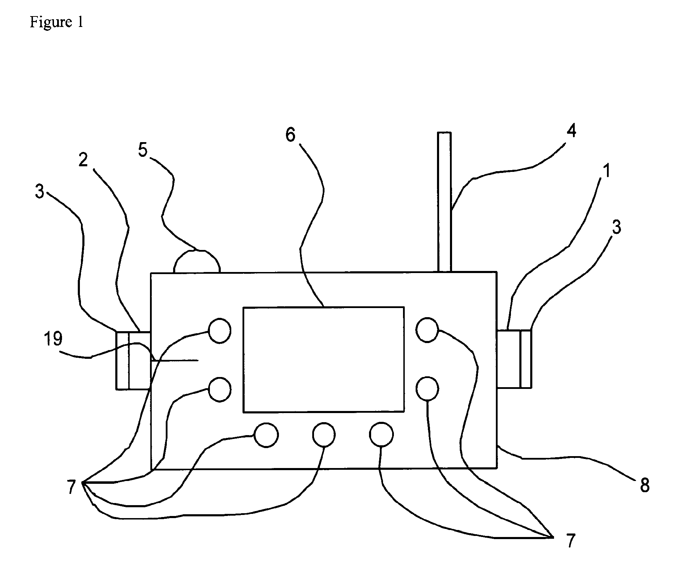

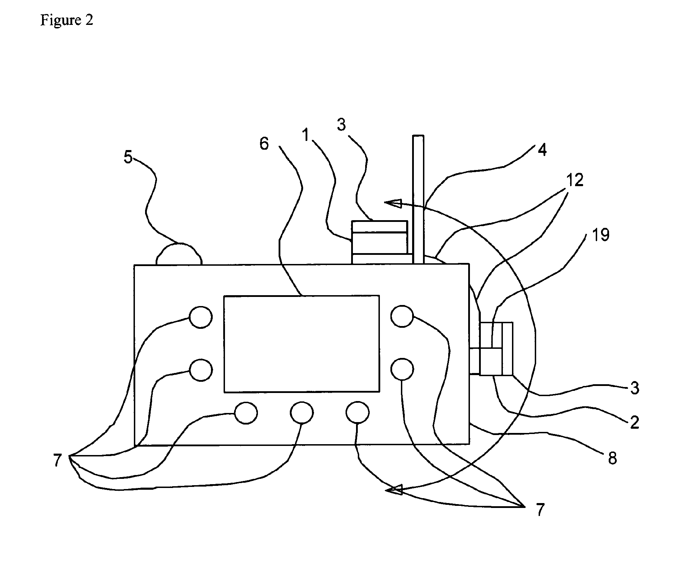

Referring to figures 1, 2 and 6, in use the operator fixes the sheet, 18, in the correct position on the part of the structure to be imaged and starts the mobile computer and its imaging program, entering data on the geometry of the structure under the sheet, 18. The operator starts two hand-held devices working in an appropriate mode using keys, 7; the sheet, 18, is attached temporarily to a substantially flat surface of a test sample, 9, to be imaged. A temporary weak adhesive can be used to hold the sheet in place on the structure. Transducers of the hand-held devices, as shown in figures 1 and 2, are pressed through the holes, 14, in the sheet, 18, onto the surface of the test sample, 9. Holes in the sheet, 14, are marked with identifying labels and the controlling program can identify these labels with the display on each hand-held device showing the label for the next hole to use. The operator selects a transducer type to use: compression or shear, then presses each hand-held device onto the sample, 9, to start a test, taking care when using shear transducers to maintain the correct plane of polarization. As the transducers are pressed against the surface a micro-switch within each device is actuated and when the micro-switches in the transmitter hand-held device and the receiver hand-held device are both actuated the transmitter transducer is excited with a swept-frequency chirp, which launches a known pattern of sound waves and/or ultrasound waves into the sample under test.

As these waves are launched the optical light transceiver, 5, sends a synchronizing pulse to the corresponding transceiver, 5, on the receiving hand-held device, causing it to start to record. The sound and/or ultrasound waves travel into the structure being inspected and are reflected and transmitted by the components therein until some waves arrive back at the receiver transducer, are converted to electrical signals, amplified and recorded. Shortly after recording the signals are processed to recognize the pattern of the swept-frequency chirp and to perform unipolar conversion and threshold detection varying in time. At this point the signal is either stored or preferably but not necessarily communicated to a nearby mobile computer upon which a synthetic aperture imaging algorithm is operating; the image is presented on the display associated with the mobile computer as already described in embodiment M and N.

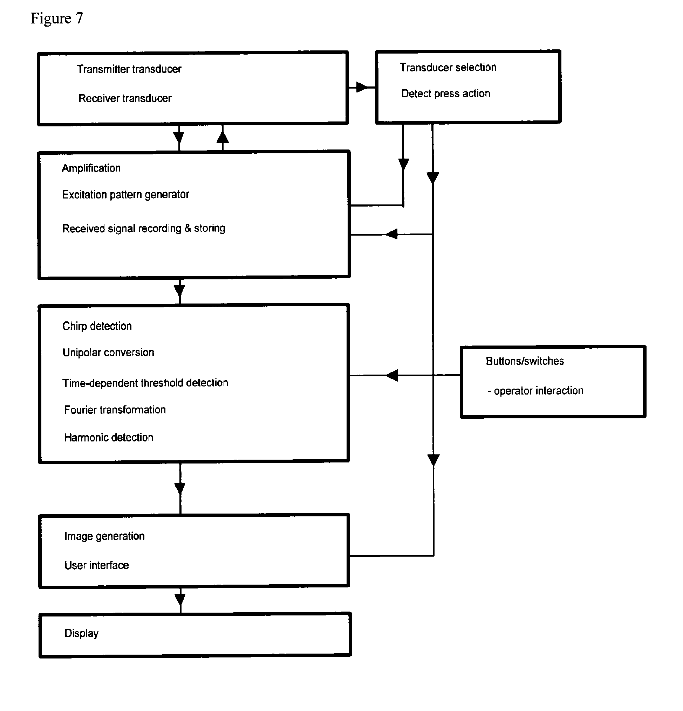

Figure 7 shows the stages of processing and communications that are, preferably but not necessarily, used by all embodiments of an inspection device as described herein, depending to some degree upon what mode has been chosen by the operator.

## Claims

1. A device for inspecting the mechanical integrity of structures, using sound waves or ultrasound waves, there is provided: (a) at least one and, preferably but not necessarily, two or more mechanically independent hand-held devices each containing one or possibly more sonic and/or ultrasonic electro-mechanical conversion transducers; (b) means for each aforementioned hand-held device to communicate with other hand-held devices, substantially without the use of cables, so that the operator or operators of the inspection device are substantially unencumbered by cables; (c) means to cause any of the transducers in either of the aforementioned hand-held devices to transmit sound waves and/or ultrasound waves; (d) means to collect electrical signals from any of the transducers, used as receivers, in the aforementioned hand-held devices, which signals are caused by the impingement of sound waves and/or ultrasound waves emerging from the structure under inspection and entering the receivers, particularly waves caused by a transmitting, hand-held device; (e) the aforementioned transducers with some or possibly all able to transmit and/or receive substantially compression sound waves or ultrasound waves, or with possibly some or possibly all of the aforementioned transducers able to generate and/or to receive substantially shear sound waves or ultrasound waves; (f) with the direction or plane of shear polarization of the aforementioned shear-transducers all being known and capable of being set by an operator; (g) each aforementioned transducer with an aperture that is preferably not larger and possibly smaller in actuating dimension than an average wavelength of sound wave and/or ultrasound wave used by an inspection device; (h) means to couple or transfer sound wave or ultrasound wave energy between each aforementioned transducer and the surface of the structure under inspection, when engaged thereupon; (i) means for creating and applying excitation patterns to transducers selected to transmit in the aforementioned hand-held devices; (j) means to synchronize the transmission of sound waves and/or ultrasound waves from one aforementioned hand-held device with the receiving of sound waves and/or ultrasound waves from another of the aforementioned hand-held devices; (k) means for collecting and storing signals received by selected transducers in one or preferably more of the aforementioned hand-held devices; (1) means in one or preferably more of the aforementioned hand-held devices for processing the signals from receivers to provide a more convenient or advantageous representation of results for the operator; (m) means in the aforementioned hand-held devices to communicate information with one or possibly more communicating devices for the purpose of improving the quality or speed of communications between hand-held devices; (n) means in the aforementioned hand-held devices to communicate information with one or possibly more processing devices for further processing or advantageous representation of results; (o) means to allow the inspection device to be used quickly and easily to inspect a structure.

2. A device for inspecting the mechanical integrity of structures, using sound waves or ultrasound waves, substantially as claimed in Claim 1 with few or preferably no electrical cables but with means for communicating between the hand-held devices that is, substantially but not exclusively, possibly radio waves, or possibly light waves or possibly more than one of these means.

3. A device for inspecting the mechanical integrity of structures, using sound waves or ultrasound waves, substantially as claimed in Claims 1 to 2 with one or possibly more communicating devices functioning as repeaters of information between any transmitter hand-held devices and any receiver hand-held devices and possibly any ancillary processing devices, with each repeater functioning so as to increase the effective range of communication or to permit communications around components of the structure or otherwise to improve the effectiveness of communications.

4. A device for inspecting the mechanical integrity of structures, using sound waves or ultrasound waves, substantially as claimed in Claims 1 to 3 with portable and lightweight hand-held devices, which are capable of performing tests quickly, preferably but not essentially within a few seconds, and continuing to work for several hours, preferably but not essentially.

5. A device for inspecting the mechanical integrity of structures, using sound waves or ultrasound waves, substantially as claimed in Claims 1 to 4 with hand-held devices having, preferably but not essentially, means to transmit either substantially compression sound and ultrasound waves or substantially shear sound and ultrasound waves and with means to receive either substantially compression sound and ultrasound waves or substantially shear sound and ultrasound waves; in this way the inspection device can be operated in as many as nine possible inspection modes according to wave-type: transmitting compression waves and receiving shear parallel waves, transmitting compression waves and receiving shear perpendicular waves, transmitting compression waves and receiving compression waves, transmitting shear parallel waves and receiving compression waves, transmitting shear parallel waves and receiving shear perpendicular waves, transmitting shear parallel waves and receiving shear parallel waves, transmitting shear perpendicular waves and receiving compression waves, transmitting shear perpendicular waves and receiving shear parallel waves and transmitting shear perpendicular waves and receiving shear perpendicular waves.

6. A device for inspecting the mechanical integrity of structures, using sound waves or ultrasound waves, substantially as claimed in Claims 1 to 5 with, preferably but not essentially, each hand-held device to be equipped with two transducers: one compression and one shear, with the shear transducer having a mark on it or on the case of the hand-held device or some other means to indicate the plane of polarization of shear.

7. A device for inspecting the mechanical integrity of structures, using sound waves or ultrasound waves, substantially as claimed in Claims 1 to 6 with hand-held devices having a compression transducer mounted at one end of a hand-held device and a shear transducer mounted at the other end of the same hand-held device so that the operator simply has to turn around a hand-held device to change between compression and shear testing.

8. A device for inspecting the mechanical integrity of structures, using sound waves or ultrasound waves, substantially as claimed in Claims 1 to 7 with hand-held devices having two transducers, one compression and one shear, mounted on a turret or other rotating means or other sliding means so that the appropriate transducer can be rotated or slid into place to change between compression and shear testing.

9. A device for inspecting the mechanical integrity of structures, using sound waves or ultrasound waves, substantially as claimed in Claims 1 to 8 with electronic means or electro-optical means or electro-magnetic means or piezoelectric means or any other means to detect the choice of transducer for transmitter or receiver for testing made by the operator and to detect when the transducer is likely to be in contact with a structure for testing, so that the choice of transducer and the contact with a structure is signalled to any electronic circuits in a hand-held device to which the transducers are connected.

10. A device for inspecting the mechanical integrity of structures, using sound waves or ultrasound waves, substantially as claimed in Claims 1 to 9 with a micro-switch or similar means to sense when a particular transducer is being pressed against a structure under test.

11. A device for inspecting the mechanical integrity of structures, using sound waves or ultrasound waves, substantially as claimed in Claims 1 to 10 with a micro-switch or similar means used to detect which transducer of two or more on a turret or rotating or sliding means is aligned for contact with a test sample.

12. A device for inspecting the mechanical integrity of structures, using sound waves or ultrasound waves, substantially as claimed in Claims 1 to 11 capable of working in a plurality of different modes, including but not necessarily limited to: transmission, transmission for tomographic imaging, reflection, reflection for synthetic aperture imaging; with the above modes compounded when the plurality of options for compression and shear wave-types as claimed herein are also included.

13. A device for inspecting the mechanical integrity of structures, using sound waves or ultrasound waves, substantially as claimed in Claims 1 to 12 with means for the positions, on the structure being inspected, of the transducers on any hand-held devices to be determined or to be defined or otherwise to be known so that the algorithms for generating images can function correctly.

14. A device for inspecting the mechanical integrity of structures, using sound waves or ultrasound waves, substantially as claimed in Claims 1 to 13 with a thin, flexible belt having a plurality of holes in a known, random or semi-random pattern on the belt or a thin flexible sheet having a plurality of holes in a known, random or semi-random pattern on the sheet that is attached to the surface of the structure to be inspected or projected optically thereupon, through the holes or projection-markings of which the transducers of any hand-held devices are inserted by an operator and thence come into contact with the surface of the structure, all with the procedures or algorithms that generate images having information about the belt or sheet being used, particularly the positions of the holes therein, so that the operator can correctly position the transducers for the experimental procedure to be efficiently managed and for correctly calculated images to be generated.

15. A device for inspecting the mechanical integrity of structures, using sound waves or ultrasound waves, substantially as claimed in Claims 1 to 14 with means to combine signals received from receiver transducers at two or more positions to create an image of the interior of the structure under test and in so doing to reduce substantially the otherwise obfuscating effect of scattering of sound waves or ultrasound waves, particularly the random scattering found in heterogeneous materials.

16. A device for inspecting the mechanical integrity of structures, using sound waves or ultrasound waves, substantially as claimed in Claims 1 to 15 with means for entering the geometrical shape of the location used for inspecting a structure and with further means for signals received from transducers at two or preferably a plurality of positions in a reflection test to be usefully combined in an image to reduce substantially the otherwise obfuscating effect of random scattering of sound waves or ultrasound waves; a preferred but not essential method for applying spatial averaging and creating an image has a focal plane chosen by an operator, divided conceptually into a plurality of focal points, possibly again chosen by the operator, and for each focal point in the focal plane as many stored echo signals as possible or desirable are processed by adding appropriate parts of them together with suitable time-delays chosen to compensate for the geometrical distances travelled by waves in the structure being inspected to create an intensity at each focal point, then by repeating the process for every focal point in the focal plane an image is developed over the entire focal plane which is relatively sharp and which is an image of the interior of the structure to be viewed by the operator.

17. A device for inspecting the mechanical integrity of structures, using sound waves or ultrasound waves, substantially as claimed in Claims 1 to 16 with means for entering the geometrical shape of the location used for inspecting a structure and with further means for signals received by transducers at two or preferably a plurality of transmission test positions through a cross-sectional plane of a structure to be combined in an image to reduce substantially the otherwise obfuscating effect of random scattering of sound waves or ultrasound waves, in which the image plane is defined by the locations of the transmitters and receivers, and conceptually divided into a plurality of image points defined by the number and locations of the transmitters and receivers; at each image point some degree of attenuation and possibly scattering of sonic or ultrasonic signals is assumed to occur and by taking the path of waves followed in each transmitter-receiver test on the structure in turn it is possible to calculate the degree of attenuation and/or the degree of scattering at each image point in the cross-section and thereby create an image to represent the geometry of the cross-section of the structure with the degree of attenuation or the degree of scattering represented thereupon using, possibly but not necessarily, a range of colours to represent the level of attenuation and/or scattering.

18. A device for inspecting the mechanical integrity of structures, using sound waves or ultrasound waves, substantially as claimed in Claims 1 to 17 using, preferably but not essentially, sound waves or ultrasound waves with wavelengths greater than the grain size of any heterogeneous material being inspected.

19. A device for inspecting the mechanical integrity of structures, using sound waves or ultrasound waves, substantially as claimed in Claims 1 to 18, in particular for inspecting structures made in part or entirely of concrete, using frequencies not substantially greater than 200 kHz.

20. A device for inspecting the mechanical integrity of structures, using sound waves or ultrasound waves, substantially as claimed in Claims 1 to 19 with means for coupling or transferring ultrasonic energy between each transducer and the surface under test, using point contact.

21. A device for inspecting the mechanical integrity of structures, using sound waves or ultrasound waves, substantially as claimed in Claims 1 to 20 with means for coupling or transferring ultrasonic energy between each transducer and the surface under test, using solid materials or substantially solid but pliable materials including: adhesives, fast-setting mortar and deformable solid materials, with, preferably but not necessarily, the transmitting surface of the transducer, the coupling agent and the material of the surface of the structure being inspected all having approximately the same value of acoustic impedance.

22. A device for inspecting the mechanical integrity of structures, using sound waves or ultrasound waves, substantially as claimed in Claims 1 to 21 with transducers with an aperture size that are not substantially greater than one wavelength of the highest frequency of sound or ultrasound used in a test.

23. A device for inspecting the mechanical integrity of structures, using sound waves or ultrasound waves, substantially as claimed in Claims 1 to 22, in particular for inspecting structures made in part or entirely of concrete, with phase coherent transducers with an aperture size that is not substantially greater than 50millimetres and preferably not substantially greater than 20millimetres.

24. A device for inspecting the mechanical integrity of structures, using sound waves or ultrasound waves, substantially as claimed in Claims 1 to 23 with transducers, preferably but not essentially, with an actuating surface, preferably but not essentially, made of a material that adheres to any coupling material or is in some other way ergonomically advantageous or compatible with any coupling agent.

25. A device for inspecting the mechanical integrity of structures, using sound waves or ultrasound waves, substantially as claimed in Claims 1 to 24 with transducers having a substantially flat transmitting surface except for a small dome or protrusion or a plurality of possible protruding shapes of characteristic size commensurate or slightly greater than the surface roughness of the surface under test, which provides means for point-contact coupling while remaining compatible for use with liquid coupling materials, adhesives and substantially solid coupling materials, so that the operator has a choice of coupling material to use to suit the prevailing test requirements and conditions of the surface to be tested.

26. A device for inspecting the mechanical integrity of structures, using sound waves or ultrasound waves, substantially as claimed in Claims 1 to 25 preferably but not essentially with means to avoid using transducers simultaneously as receivers and transmitters thereby retaining maximum information from the interior of the structure under test.

27. A device for inspecting the mechanical integrity of structures, using sound waves or ultrasound waves, substantially as claimed in Claims 1 to 26 with means to drive transmitter transducers with electrical excitation in a controlled pattern, which pattern is chosen to suit the requirements of the inspection, the material under test and capabilities of the transducers, in particular the useful frequency bandwidth but otherwise, preferably but not essentially, covering as wide a range of frequencies as possible.

28. A device for inspecting the mechanical integrity of structures, using sound waves or ultrasound waves, substantially as claimed in Claims 1 to 27 with means to drive transmitter transducers with an electrical excitation pattern substantially in the form of swept-frequency chirps, comprising bursts of sine waves, with each burst lasting a time not less than one cycle of the shortest cycle of the burst of sine waves, during which burst-time the frequency of the sine wave is changed or swept.

29. A device for inspecting the mechanical integrity of structures, using sound waves or ultrasound waves, substantially as claimed in Claims 1 to 28, in particular for inspecting structures made in part or entirely of concrete, with means to drive transmitter transducers with swept-frequency chirps of duration in the range 30 to 300 microseconds, within which time and the frequency sweeps between two frequencies somewhere in the range 200 kHz to 10 kHz.

30. A device for inspecting the mechanical integrity of structures, using sound waves or ultrasound waves, substantially as claimed in Claims 1 to 29, in particular for inspecting structures made in part or entirely of concrete, with means to process signals from receiver transducers to detect occurrences of any pattern sent out by transmitter transducers and to provide an event marker with more precise timing than the duration of the original pattern.

31. A device for inspecting the mechanical integrity of structures, using sound waves or ultrasound waves, substantially as claimed in Claims 1 to 30 with means to process signals from receiver transducers to detect occurrences of patterns sent from transmitter transducers, in which the processing means is a signal processing algorithm sometimes called matched filtering, arguably a form of mathematical cross-correlation, and which uses as the basis of the pattern recognition either a time-reversed copy of the electrical excitation signal pattern or a time-reversed part of a recording of a pattern transmitted in a calibrating test on a suitable material using substantially the same transducers as are used in the inspection device on the sample under test, so that any modifications to or distortions of the pattern, either caused by the transmitter or by the receiver, can be included in the pattern to be recognized and thereby contribute to a sharper or more precise detection of the event.

32. A device for inspecting the mechanical integrity of structures, using sound waves or ultrasound waves, substantially as claimed in Claims 1 to 31 with matched filtering or other pattern recognition means to process signals from receiver transducers to detect occurrences of any kind of regular excitation used to drive transmitter transducers, in particular including sharp spike excitation, which results in received signals with characteristic patterns that can be recorded in a calibration experiment and using that part of such a recording containing the pattern as the basis of the matched filter or other pattern recognition means.

33. A device for inspecting the mechanical integrity of structures, using sound waves or ultrasound waves, substantially as claimed in Claims 1 to 32 with means to process signals, preferably but not essentially after pattern recognition, by converting them from a bipolar form into a unipolar form, preferably but not necessarily, using the magnitude of the mathematical analytic function of the signal or possibly simple rectification or possibly taking the magnitude of the signal or other possible processing means, the latter two specific methods and possibly others, preferably but not essentially, followed by low-pass or band-pass filtering to smooth the signal.

34. A device for inspecting the mechanical integrity of structures, using sound waves or ultrasound waves, substantially as claimed in Claims 1 to 33 with means to process signals, preferably but not essentially after unipolar conversion, to detect peaks in the signal using a time-varying threshold based upon the statistics of one or more signals received from substantially similar random scattering materials as those that may be in the structure under test and, preferably but not essentially, by adjusting the parameters of the statistics to provide control over the sensitivity of detection of peaks of interest.

35. A device for inspecting the mechanical integrity of structures, using sound waves or ultrasound waves, substantially as claimed in Claims 1 to 34 with means to process signals, preferably but not essentially after unipolar conversion, to detect peaks in the signal using a time-varying threshold, preferably but not necessarily, based upon the Weibull or Rayleigh mathematical distributions and by adjusting the parameters of the chosen distribution to provide control over the sensitivity of detection of peaks of interest.

36. A device for inspecting the mechanical integrity of structures, using sound waves or ultrasound waves, substantially as claimed in Claims 1 to 35 with means, preferably but not essentially, used after the process of pattern recognition and possibly but not necessarily after unipolar-conversion to calculate the magnitude of the Fourier transform of the processed signal and to display the resulting frequency domain representation of the signal for the operator to determine any peaks therein, thereby to help the operator to establish the presence of delamination cracks in the structure under inspection.

37. A device for inspecting the mechanical integrity of structures, using sound waves or ultrasound waves, substantially as claimed in Claims 1 to 36 with means, preferably but not essentially, to add together either time-domain signals possibly with time shifts or to add together frequency-domain signals from nearby test locations to help suppress the effect of random grain scattering in the resultant signal and to emphasize the effect of systematic features in the resultant signal.

38. A device for inspecting the mechanical integrity of structures, using sound waves or ultrasound waves, substantially as claimed in Claims 1 to 37 with means to process signals from receivers to recognize therein the pattern of any harmonics or sub-harmonics of the excitation pattern used to drive transmitter transducers and means to use the harmonic or sub-harmonic signal for all measurements normally done with the fundamental pattern.

39. A device for inspecting the mechanical integrity of structures, using sound waves or ultrasound waves, substantially as claimed in Claims 1 to 38 with communicating means to synchronize accurately the excitation of the transmitter in one hand-held device with the start of recording of a signal derived from a receiver in either the same or another hand-held device, in particular having communicating means with low jitter or variability in the start of the recording, so that the jitter is substantially smaller than the period of the highest frequency transmitted by a hand-held device, if the transmitter and receiver are not in the same hand-held device, when two or more hand-held devices are used for inspection, it is preferable but not essential to use light or similar communicating means to convey a synchronizing signal between hand-held devices in an inspection device, however, communicating means using radio are also possible and one form of radio communication that is possible is to use is the natural electro-magnetic wave generated when the transmitter is actuated, which has approximately the same pattern as the electrical drive signal to the transmitter transducer so it is possible for the receiving hand-held device to be synchronized from this signal, generally present in the signal from a receiver transducer, particularly if pattern recognition means are used to process the received electro-magnetic signal directly without first recording any signal, since the result of pattern recognition is a sharper event pulse; all synchronizing means being preferably but not essentially used with averaging of measured values or signals taken over two or a plurality of inspection tests so as to reduce errors in those values or signals.

40. A device for inspecting the mechanical integrity of structures, using sound waves or ultrasound waves, substantially as claimed in Claims 1 to 39 with a ping-pong mode of operation which uses synchronizing signals between two hand-held devices, referred to here as A and B, derived from the sound waves or ultrasound waves transmitted as a part of an inspection test, in which mode of operation hand-held device A transmits a known pattern of sound waves or ultrasound waves into the structure being inspected and starts recording from the same transducer at the same time or possibly at some known, delayed time thereafter, with the waves travelling through the structure to the receiver of hand-held device B, which uses its pattern recognition means directly without recording to detect occurrences of the transmitted pattern of waves whereupon hand-held device B changes from receiving to transmitting, using a known pattern of excitation, preferably but not necessarily a different pattern of excitation from that used by hand-held device A, possibly but not necessarily a time-reversed version of the pattern transmitted by hand-held device A, with the re-transmitted pattern of waves from hand-held device B travelling back through the structure to be collected and processed by hand-held device A, now operating as a receiver; improvements in accuracy to the preceding method can be achieved by touching the transducers of the two hand-held devices directly or preferably by touching them against opposing sides of a test block, through which waves travel in a known time, any time delay caused by the ping-pong method can be calculated from this correcting experiment and subtracted from the total time measured in subsequent inspection tests of this kind, after subtracting time delays the resulting time is divided by two to get the time taken to pass once through the distance in the structure travelled by the waves, then speed is calculated by dividing the distance travelled through the structure by the final, corrected time result; amplitude values derived from ping-pong mode tests can also be used for imaging; by performing two or a plurality of inspection tests in ping-pong mode and by calculating the average result it is possible to reduce the error due to the higher jitter that may result from using the ping-pong mode.

41. A device for inspecting the mechanical integrity of structures, using sound waves or ultrasound waves, substantially as claimed in Claims 1 to 40 with, preferably but not essentially, one or possibly a plurality of data processing devices in any hand-held device, preferably but not necessarily: a computer or a microprocessor or a digital signal processor or a microcontroller or combinations of the aforementioned devices along with suitable memory and other electrical circuits; the data processing devices, preferably but not necessarily, control some or all of the following functions: the transmission of any sound and ultrasonic waves, the receiving of any sound or ultrasound waves, the conversion of any received waves into electrical signals and then into digital signals, recording of signals, the processing of signals, the communication with other hand-held devices, the communication with any other communication devices or processing devices, the creation of images, responding to button presses by the operator.

42. A device for inspecting the mechanical integrity of structures, using sound waves or ultrasound waves, substantially as claimed in Claims 1 to 41 with, preferably but not essentially, any electronic circuits and other sensitive components in any hand-held device held inside an enclosure, preferably but not essentially, an enclosure sealed against moisture, humidity, dust and other environmental agents that might disturb the normal operation of the inspection device.

43. A device for inspecting the mechanical integrity of structures, using sound waves or ultrasound waves, substantially as claimed in Claims 1 to 42 with any hand-held device, preferably but not necessarily, containing a source of electrical energy, possibly but not necessarily, a re-chargeable battery or batteries. Each hand-held device is, preferably but not essentially, sufficiently lightweight to be carried by the operator in the operator's hand.

44. A device for inspecting the mechanical integrity of structures, using sound waves or ultrasound waves, substantially as claimed in Claims 1 to 43 with means for the operator to control a test using, possibly but not necessarily, switches or similar devices, preferably but not necessarily, mounted on each hand-held device in conjunction with information on a display panel mounted, preferably but not necessarily, on each hand-held device.

45. A device for inspecting the mechanical integrity of structures, using sound waves or ultrasound waves, substantially as claimed in Claims 1 to 44 with means in one or more hand-held devices to calculate the speed of sound or ultrasound of compression and/or shear waves in the structure under test and with further means to enter the value of a dimension through which the waves have travelled in the structure under inspection into one or possibly more hand-held devices so that the calculation of speed can be done.

46. A device for inspecting the mechanical integrity of structures, using sound waves or ultrasound waves, substantially as claimed in Claims 1 to 45 with, possibly, a separate, ancillary display panel having, preferably but not essentially, better means to display images than any display panel on any hand-held devices and with, preferably but not necessarily, an ancillary processor in a processing device, possibly but not necessarily a mobile computer for faster image processing and, possibly but not essentially, means for storing of information.

47. A device for inspecting the mechanical integrity of structures, using sound waves or ultrasound waves, substantially as claimed in Claims 1 to 46 with an ancillary data entry and control device, possibly but not necessarily, a mobile computer and for the ancillary device either to communicate the settings and data to the hand-held devices forming the inspection device or used the settings and data locally for processing connected with the inspection device.

48. A device for inspecting the mechanical integrity of structures, using sound waves or ultrasound waves, substantially as claimed in Claims 1 to 47 with transducers on any hand-held devices that can be detached and replaced but otherwise held in place by mechanical means.

49. A device for inspecting the mechanical integrity of structures, using sound waves or ultrasound waves, substantially as claimed in Claims 1 to 48 with means, preferably but not essentially, for each hand-held device in an inspection device to be capable of use interchangeably, either as a transmitter or as a receiver.

50. A device for inspecting the mechanical integrity of structures, using sound waves or ultrasound waves, substantially as claimed in Claims 1 to 49 and substantially as described hereinbefore, especially with reference to one or more of the Figures shown in the accompanying drawings.
